Europäisches Patentamt

European Patent Office

Office européen des brevets

(19)

(11) Veröffentlichungsnummer: **0 100 406 B1**

## (12) EUROPÄISCHE PATENTSCHRIFT

(45) Veröffentlichungstag der Patentschrift:
06.03.85

(21) Anmeldenummer: 83105112.3

(22) Anmeldetag: 24.05.83

(51) Int. Cl.⁴: **C 07 C 31/08, C 07 C 29/136**

(54) Verfahren zur kontinuierlichen Herstellung von Ethanol.

(30) Priorität: 04.06.82 DE 3221077

(43) Veröffentlichungstag der Anmeldung:
15.02.84 Patentblatt 84/7

(45) Bekanntmachung des Hinweises auf die Patenterteilung:
06.03.85 Patentblatt 85/10

(84) Benannte Vertragsstaaten:
BE DE FR GB IT NL

(56) Entgegenhaltungen:
DE - A - 1 921 848
DE - A - 2 321 101
US - A - 3 478 112

(73) Patentinhaber: BASF Aktiengesellschaft,
Carl-Bosch-Strasse 38, D-6700 Ludwigshafen (DE)

(72) Erfinder: Schuster, Ludwig, Dr., Weinheimer Strasse 44,
D-6703 Limburgerhof (DE)
Erfinder: Mueller, Franz-Josef, Dr.,
Mueller-Thurgau-Weg 1, D-6706 Wachenheim (DE)
Erfinder: Anderlohr, Axel, Dr., Bruehler Ring 25,
D-6800 Mannheim (DE)
Erfinder: Blei, Peter, Berliner Strasse 16, D-6702 Bad
Duerkheim (DE)
Erfinder: Eigenberger, Gerhart, Dr., Deidesheimer
Strasse 21, D-6730 Neustadt (DE)
Erfinder: Hoeppner, Bernd, Dr., Muehlweg 24,
D-6730 Neustadt (DE)
Erfinder: Kaibel, Gerd, Robert-Bosch-Strasse 4,
D-6840 Lampertheim (DE)
Erfinder: Steiner, Wolfgang, Dr., Haupstrasse 38,
D-6701 Friedelsheim (DE)

**Beschreibung**

Die vorliegende Erfindung betrifft ein verbessertes Verfahren zur kontinuierlichen Herstellung von Ethanol durch katalytische Hydrierung von Essigsäure mit Wasserstoff unter erhöhtem Druck und erhöhter Temperatur.

Es ist allgemein bekannt, Carbonsäuren katalytisch zu den entsprechenden Alkoholen zu hydrieren, jedoch ist die Hydrierung von Essigsäure bisher noch nicht befriedigend gelungen.

Verwendet man hierzu Ruthenium-Katalysatoren (US-PS 2 607 807 und die entsprechende Arbeit in J. Chem. Soc. Band 77, 1955, S. 3766), so betragen die Ausbeuten trotz des extrem hohen Drucks von 700 bis 950 bar höchstens 88% und bei dem geringeren Druck von 200 bar ist die Ausbeute von 41% für technische Zwecke indiskutabel.

Ebenso unbefriedigend verläuft die Reaktion mit Rhenium-Katalysatoren (J. Org. Chem. Band 23, 1959, S. 1850). Die Ethanolausbeute beträgt hier günstigenfalls nur rd. 40% (59%, bezogen auf 68% Umsatz) und muß zudem mit der unwirtschaftlich langen Verweilzeit von 60 Stunden erkauft werden. Verschärft man die Reaktionsbedingungen zugunsten technisch sinnvoller Verweilzeiten, so sinkt die Ausbeute auf 16%.

Sieht man von den mangelhaften Ergebnissen bei den vorgenannten Verfahren ab, so sind diese auch deswegen nachteilig, weil sie teure Katalysatoren erfordern. Die Entwicklung auf dem Gebiet der Carbonsäurehydrierung erstreckte sich daher in der Folgezeit auf die Verwendung billigerer Kontakte, darunter vor allem solcher Katalysatoren, die Cobalt als wesentlichen Bestandteil enthalten. Hiermit lassen sich zwar höhere Carbonsäuren erfolgreich zu den Alkoholen hydrieren, nicht aber die Essigsäure, wie aus der einschlägigen Patentliteratur indirekt hervorgeht. So betrifft die DE-PS 1 235 879 allgemein die Hydrierung von Carbonsäuren unter erhöhtem Druck und bei erhöhter Temperatur in Gegenwart von Cobalt enthaltenden Katalysatoren, jedoch ist die Hydrierung von Essigsäure in keinem der vierzehn Ausführungsbeispiele beschrieben.

Ähnlich verhält es sich mit dem Verfahren der DE-OS 1 921 848 — kontinuierliche Hydrierung von Carbonsäuren mit Cobalt-Katalysatoren bei 135 bis 350°C — deren experimenteller Teil ausschließlich die Hydrierung der Pelargonsäure zu Nonanol betrifft, und auch das Verfahren der DE-OS 2 321 101 — Verwendung von Co/Mo-Katalysatoren — wird lediglich für den Fall der Hydrierung eines Gemisches aus Hydroxycapronsäure und Adipinsäure näher erläutert.

Wenngleich die Essigsäure in den drei letztgenannten Patentveröffentlichungen erwähnt ist, so ist ihre Hydrierung doch nicht experimentell belegt. Der Grund hierfür ist nicht angegeben, stellt sich aber schnell heraus, wenn man versucht, die Essigsäure nach den allgemeinen Vorschriften der Patentveröffentlichungen zu hydrieren: die Essigsäure ist nämlich unter den erforderlichen scharfen Reaktionsbedingungen so korrosiv, daß sie den Cobalt-Kontakt binnen kurzem zerstört.

Der Erfindung lag daher die Aufgabe zugrunde, die Essigsäurehydrierung mittels Cobalt enthaltender Katalysatoren unter Vermeidung dieses Nachteils zu verbessern und damit technisch-wirtschaftlich nutzbar zu machen.

Demgemäß wurde ein kontinuierliches Verfahren zur Herstellung von Ethanol durch Hydrierung von Essigsäure unter erhöhtem Druck und bei erhöhter Temperatur gefunden, welches dadurch gekennzeichnet ist, daß man hierzu einen überwiegend Cobalt enthaltenden Katalysator als Kontakt verwendet und daß man Essigsäure und Wasserstoff im Bereich von 210 bis 330°C und 10 bis 350 bar unter solchen Bedingungen durch den Reaktor leitet, daß sich hierbei keine flüssige Phase ausbildet.

Die Erfindung beruht auf der überraschenden Beobachtung, daß die an sich äußerst aggressive Essigsäure ihre korrosive Eigenschaft in der Gasphase praktisch vollständig verliert. Das Verfahren läßt infolgedessen nicht nur den Kontakt unbeschädigt, sondern es macht auch die Verwendung teurer Metalle oder Metall-Legierungen als Materialien für den Reaktor und Reaktorzubehör entbehrlich.

Die Maßgabe, die Hydrierung unter Vermeidung einer Flüssigphase vorzunehmen, bedeutet, daß Druck und Temperatur in einem bestimmten funktionalen Zusammenhang stehen müssen. Da in diese Funktion außerdem noch die Konzentrationen aller Reaktionspartner — Wasserstoff, Essigsäure, Ethanol, Wasser-Ethylacetat — eingehen, die im Rahmen der Betriebsweise mit und ohne Rückführung des Reaktoraustrages ihrerseits variabel sind, ist es schwierig, Druck und Temperatur zu berechnen, zumal die Ausbildung einer Flüssigphase auch vom Strömungszustand des Gasgemisches abhängt. Statt dessen empfehlen sich einige Vorversuche mit einer Versuchsapparatur, welche es gestattet, den Aggregatzustand des Reaktionsgemisches zu beobachten.

Da die Reaktionsgeschwindigkeit mit steigendem Druck und steigender Temperatur zunimmt, arbeitet man vorzugsweise im Bereich von 40 bis 120 bar und 230 bis 270°C. Oberhalb von etwa 280°C macht sich die Bildung von Kohlenwasserstoffen wie hauptsächlich Methan bemerkbar. Zumindest im oberen Druck- und Temperaturbereich liegt das Reaktionsgut in überkritischem Zustand vor, so daß sich eine Flüssigphase nicht mehr bilden kann.

Die zu verwendenden Katalysatoren sollen überwiegend, also mindestens zu 50 Gew.-%, aus Cobalt bestehen. Reines Cobalt erfüllt zwar auch seinen Zweck, jedoch haben sich weitere Katalysatorkomponenten wie Kupfer, Mangan, Chrom, Molybdän und Phosphorsäure als vorteilhaft erwiesen.

Man stellt die Katalysatoren, die an sich z. B.

aus der DE-PS 1 235 879 bekannt sind, in üblicher Weise aus dem entsprechenden Gemisch der Metalloxide und gegebenenfalls weiterer Komponenten, z. B. Phosphorsäure, her, indem man diese Gemische einige Stunden lang im Wasserstoffstrom erhitzt, wobei die Oxide größtenteils zu den Metallen reduziert werden. Neben den aktiven Bestandteilen können die Katalysatoren auch noch sonstige, im wesentlichen inerte Materialien wie Aluminiumoxid oder Kieselsäure enthalten.

Man kann die Katalysatoren als Trägerkatalysatoren oder vorzugsweise als Substanzkatalysatoren einsetzen: Trägerkatalysatoren werden zweckmäßigerweise durch ein- oder mehrmaliges Imprägnieren des Trägermaterials — vorzugsweise Aluminiumoxid oder Kieselsäure — mit einer wäßrigen Lösung der Metallsalze, vorzugsweise der Nitrate, hergestellt. Nach dem Imprägnieren und Trocknen wird die Masse erhitzt, wobei die Metallsalze in die Metalloxide übergehen, und anschließend werden die Oxide mit Wasserstoff wie im Falle der Substanzkatalysatoren größtenteils zu den Metallen reduziert.

Gut geeignete Katalysatoren sind beispielsweise solche, in denen die Mengen der aktiven Komponenten aus folgenden Bereichen gewählt sind:

50 bis 80 Gew.-% Co
10 bis 30 Gew.-% Cu
0 bis 10 Gew.-% Mn
0 bis 5 Gew.-% Mo
0 bis 5 Gew.-% Phosphorsäure

Die für die Hydrierung erforderliche Menge des Katalysators hängt von den Reaktionsbedingungen und von dessen Aktivität ab, die ihrerseits eine Funktion der Teilchengröße und der Oberfläche ist. Für eine strömungsgünstige Festbettschüttung empfehlen sich Teilchen von 2 bis 5 mm Höhe und Durchmesser, sei es in Form von Substanzkatalysatoren oder in Form von Trägerkatalysatoren mit einem Gehalt von 10 bis 20 Gew.-% an aktiver Masse.

Mit einer Schüttung von 1 l derartiger Teilchen lassen sich bei 250"C und 100 bar pro Stunde etwa 0,1 bis 0,7 kg Essigsäure hydrieren.

Als Reaktor kann ein Rohrreaktor oder, wegen der besseren Temperaturführung, vorzugsweise ein Rohrbündelreaktor verwendet werden. Die recht erhebliche Reaktionswärme kann im Falle des Rohrbündelreaktors durch Außenkühlung und im Falle des Rohrreaktors durch Innenkühlung mittels Kühlschlangen abgeführt werden. Verwendet man einen Rohrreaktor, kann es sich auch empfehlen, das Einsatzstoffgemisch aus Wasserstoff und Essigsäure mit dem Reaktoraustrag zu verdünnen, welchem in einem üblichen externen Kreislauf die Wärme entzogen wird. Hierbei beträgt das Gewichtsverhältnis der rückgeführten Stoffe zu frischer Essigsäure vorzugsweise 1 : 1 bis 2 : 1.

Vorzugsweise führt man Wasserstoff und Essigsäure im Gleichstrom durch den Reaktor.

Hierbei empfiehlt es sich, die Essigsäure gasförmig in den Reaktor zu geben. Allerdings ist auch eine flüssige Zuführung möglich, da die Essigsäure unter den Reaktionsbedingungen augenblicklich verdampft und hierdurch bereits einen Teil der Reaktionswärme aufnimmt. Da der Katalysator andererseits nicht mit flüssiger Essigsäure in Berührung kommen soll, ist es im letztgenannten Fall zweckmäßig, den Reaktor nicht gänzlich mit dem Kontakt zu füllen. Der Abstand des Kontaktes zur Einlaßstelle der Essigsäure ergibt sich aus der Verdampfungsgeschwindigkeit und der Strömungsgeschwindigkeit.

Als Reaktormaterialien eignen sich normale Edelstähle, z. B. ®V-Stähle.

Das erfindungsgemäße Verfahren gestattet die praktisch vollständige Hydrierung der Essigsäure, wobei man das Ethanol in über 95%iger Ausbeute erhält. Daneben erhält man noch etwas Ethylacetat, geringe Mengen sonstiger Nebenprodukte sowie rd. 30 Gew.-% Wasser, bezogen auf die Essigsäure. Die Aufarbeitung des Reaktionsgemisches auf Ethanol kann wie üblich vorgenommen werden.

Im Rahmen der Bestrebungen, Ethanol aus Methanol, Kohlenmonoxid und Wasserstoff zu gewinnen, stellt das erfindungsgemäße Verfahren die erfolgreiche Bewältigung des kritischen Teilschrittes der Essigsäurehydrierung dar.

## Beispiel

Durch einen mit Katalysator gefüllten Versuchsreaktor von 2 m Höhe und 4 cm lichter Weite, der mit Edelstahl ausgekleidet war, wurden bei 250°C und einem Gesamtdruck von 300 bar (Wasserstoff-Partialdruck ca. 270 bar) stündlich 0,5 kg Eisessig hydriert, der zusammen mit 2,5 Nm dem Wasserstoff von oben in den Reaktor gegeben wurde. Beim Eintritt in den Reaktor verdampfte die flüssig eingeleitete Beschickung augenblicklich, so daß am Katalysatorbett keine flüssige Phase entstehen konnte. Wie in einer Modellapparatur, die mit einem Sichtfenster versehen war, festgestellt wurde, kann sich unter den angegebenen Reaktionsbedingungen keine Flüssigphase ausbilden.

Die austretenden Gase wurden unter Druck in einem externen Kühler gekühlt, wonach pro Stunde rd. 520 g flüssiges Reaktionsgemisch anfielen. Dieses Gemisch bestand aus

68,2 Gew.-% Ethanol
29,0 Gew.-% Wasser
1,4 Gew.-% Ethylacetat
0,5 Gew.-% n-Butanol
0,2 Gew.-% Essigsäure und
0,7 Gew.-% sonstigen Produkten

Dies entspricht einer Ethanolausbeute von 97%, die über die Versuchsdauer von 30 Tagen unverändert blieb. Dies bedeutet, daß die Aktivität des Katalysators bei der erfindungsgemäßen Betriebsweise konstant blieb.

Die Katalysatorschüttung hatte ein Volumen von rd. 1,6 l und reichte im Reaktor etwa 130 cm hoch. Der Katalysator, der aus Strangpreßlingen von 4 mm Durchmesser und 3 bis 6 mm Höhe bestand, wurde vor der Hydrierung in die aktive Form überführt. Hierzu wurden 3950 g der oxidischen Teilchen, ($CoO$, $CuO$, $Mn_3O_4$ und $MoO_3$) 48 Stunden lang in einem $H_2/N_2$-Strom auf 350°C erhitzt, wobei der Katalysator größtenteils in die metallische Form überging. Bezogen auf den Metallgehalt setzte sich der Katalysator aus 71 Gew.-% Co, 18,5 Gew.-% Cu, 7,5 Gew.-% Mn und 3 Gew.-% Mo zusammen.

Vergleichsversuch

Bei einer Temperatur von 200°C und einem Druck von 400 bar, aber sonst gleichen Bedingungen mußte der Modellbetrachtung zufolge Flüssigphase zugegeben sein. Die Ethanolausbeute betrug in diesem Falle nur 81%, sank aber bereits im Laufe von 5 Tagen auf 63%.

## Patentansprüche

1. Verfahren zur kontinuierlichen Herstellung von Ethanol durch Hydrierung von Essigsäure unter erhöhtem Druck und bei erhöhter Temperatur, dadurch gekennzeichnet, daß man hierzu einen überwiegend Cobalt enthaltenden Katalysator als Kontakt verwendet und daß man Essigsäure und Wasserstoff im Bereich von 210 bis 330°C und 10 bis 350 bar unter solchen Bedingungen durch den Reaktor leitet, daß sich hierbei keine flüssige Phase ausbildet.

2. Verfahren nach Anspruch 1, dadurch gekennzeichnet, daß der Druck 40 bis 120 bar und die Temperatur 230 bis 270°C beträgt.

3. Verfahren nach den Ansprüchen 1 und 2, dadurch gekennzeichnet, daß die aktive Masse des Katalysators zu mindestens 50 Gew.-% aus Cobalt, als Metall gerechnet, besteht.

4. Verfahren nach Anspruch 3, dadurch gekennzeichnet, daß der Katalysator neben Cobalt als weitere aktive Bestandteile Kupfer, Mangan, Molybdän und/oder Chrom (jeweils als Metall gerechnet) und/oder Phosphorsäure enthält.

## Claims

1. A process for the continuous preparation of ethanol by hydrogenating acetic acid under superatmospheric pressure and at elevated temperature, wherein a predominantly cobalt-containing catalyst is used and acetic acid and hydrogen are passed through the reactor, at from 210 to 330°C and under from 10 to 350 bar, under such conditions that a liquid phase is not formed during this procedure.

2. A process as claimed in claim 1, wherein the pressure is from 40 to 120 bar and the temperature is from 230 to 270°C.

3. A process as claimed in claims 1 and 2, wherein the active catalytic material contains not less than 50% by weight, calculated as metal, of cobalt.

4. A process as claimed in claim 3, wherein the catalyst contains, in addition to cobalt, as further active components, copper, manganese, molybdenum and/or chromium (calculated as metal in each case) and/or phosphoric acid.

## Revendications

1. Procédé de production en continu de l'éthanol par hydrogénation de l'acide acétique sous température et pression accrues, caractérisé en ce que la réaction est effectuée en présence d'un catalyseur avec une teneur prépondérante en cobalt et l'on fait passer l'acide acétique et l'hydrogène dans la gamme de 210 à 330°C et de 10 à 350 bars à travers le réacteur dans des conditions telles qu'il n'y ait pas formation d'une phase liquide.

2. Procédé suivant la revendication 1, caractérisé en ce que la pression est comprise entre 40 et 120 bars et la température entre 230 et 270°C.

3. Procédé suivant la revendication 1 ou la revendication 2, caractérisé en ce que la matière active du catalyseur est constituée à raison d'au moins 50% en poids de cobalt, calculé comme métal.

4. Procédé suivant la revendication 3, caractérisé en ce que le catalyseur contient, à côté du cobalt, d'autres ingrédients actifs choisis parmi le cuivre, le manganèse, le molybdène et(ou) le chrome (calculés comme métaux) et(ou) l'acide phosphorique.